# EUROPÄISCHE PATENTSCHRIFT

(11) **EP 4 216 854 B1**
(45) Veröffentlichungstag und Bekanntmachung des Hinweises auf die Patenterteilung: **11.12.2024**
(21) Anmeldenummer: 21786351.3
(22) Anmeldetag: 24.09.2021
(51) Int. Cl.: A61B 18/12, A61B 90/00, A61B 18/00, A61B 17/34, A61B 18/14, A61B 10/02, A61B 10/04

(54) **MEDIZINISCHES INSTRUMENT ZUR MINIMALINVASIVEN ENTNAHME VON BIOLOGISCHEM MATERIAL AUS EINEM MENSCHLICHEN ODER TIERISCHEN KÖRPER**
MEDICAL INSTRUMENT FOR MINIMALLY INVASIVE REMOVAL OF BIOLOGICAL MATERIAL FROM A HUMAN OR ANIMAL BODY
INSTRUMENT MÉDICAL POUR LE PRÉLÈVEMENT DE MATÉRIEL BIOLOGIQUE SUR UN CORPS HUMAIN OU ANIMAL PAR VOIE MINI-INVASIVE

(30) Priorität: 24.09.2020 DE 102020124917
(43) Veröffentlichungstag der Anmeldung: 02.08.2023
(73) Patentinhaber: Schure, Frank, 16359 Biesenthal (DE)
(72) Erfinder: SCHURE, Viola, 16359 Biesenthal (DE); SCHURE, Frank, 16359 Biesenthal (DE)
(74) Vertreter: Heinemeyer, Karsten
(86) Internationale Anmeldenummer: PCT/EP2021/076414
(87) Internationale Veröffentlichungsnummer: WO 2022/064013

(56) Entgegenhaltungen:
- EP-A1- 3 366 232
- EP-A2- 1 864 622
- WO-A1-01/89388
- WO-A2-99/04704
- DE-A1- 102017 011 054
- US-A- 4 905 691
- US-A- 5 573 008
- US-A1- 2001 014 778
- US-A1- 2016 151 085

## Beschreibung

Die Erfindung betrifft ein medizinisches Instrument zur minimalinvasiven Entnahme von biologischem Material aus einem menschlichen oder tierischen Körper nach Anspruch 1.

Es sind eine Vielzahl unterschiedlicher medizinischer Instrumente für endoskopische Eingriffe, insbesondere Biopsienadeln bzw. Biopsiesonden, bekannt, mit denen biologisches Material, wie Tumore oder Teile von Geweben oder Organen, aus dem menschlichen oder tierischen Körper entfernt werden können. In Bezug auf die Entfernung von Tumoren ist es in diesem Zusammenhang bekannt, diese zu stanzen und nur einen Teil für eine anschließende pathologische Untersuchung zu entfernen oder aber einen Tumor mit Hilfe einer Vakuumbiopsie vollständig zu entfernen. Da der Tumor während der Vakuumbiospie allerdings zerschnitten und in kleinen Fraktionen abgesaugt wird, ist es bei Einsatz dieses Biopsieverfahrens nicht möglich, die Resektionsgrenzen des Tumors exakt pathologisch aufzuarbeiten. Erkenntnisse über die ursprüngliche Form, Ausrichtung und Struktur des Tumors innerhalb des Körpers können daher mit diesem Verfahren nicht gewonnen werden. Im Gegensatz zu den beiden zuvor genannten Verfahren ist es bislang nur mit einer offenen Biopsie, die gestützt durch ein bildgebendes Verfahren durchgeführt wird, möglich, einen Tumor vollständig und in seiner ursprünglichen Gestalt aus dem Körper eines Patienten zu entfernen.

Aus der EP 0 761 170 A1 ist eine Vorrichtung zum Entfernen von Gewebe aus einem menschlichen Körper bekannt. Die beschriebene Vorrichtung verfügt über einen länglichen Grundkörper mit einer Öffnung am distalen Ende und mit einem Hohlraum zur Aufnahme von ausgeschnittenem Gewebe. Ferner ist ein Schneidelement vorgesehen, das während des Eingriffs quer zum länglichen Grundkörper in unmittelbarer Nähe der Öffnung bewegt werden kann, sodass entfernte Gewebeteile in den Hohlraum gefördert werden. Nach Entfernung des entsprechenden Materials kann der mit Gewebe gefüllte Aufnahmeraum durch einen stumpfen Verschluss verschlossen werden.

Im Weiteren beschreibt die US 5 353 804 A eine Vorrichtung zum Entfernen von Mammatumoren. Die Vorrichtung verfügt im Wesentlichen über ein innerhalb einer Kanüle in Längsrichtung bewegbares Schneidelement, das an seinem distalen Ende eine Schneidkante aufweist. Ferner ist im Inneren des Schneidelements und koaxial dazu angeordnet ein Draht vorgesehen, der bis in einen auf der dem Schneidelement gegenüberliegenden Seite in den Gewebebereich geführt und hier mit einem Ankerelement fixiert wird. Mit Hilfe der Fixierung durch das Ankerelement soll eine sichere, möglichst zielgenaue Biopsie des Tumors ermöglicht werden.

Die DE 38 16 477 A1 beschreibt eine Vorrichtung zur Entnahme von biologischem Material aus einem menschlichen Körper mit einem Schaft, der an einem Ende eine schlingenförmige Entnahmeeinrichtung aufweist. Diese Entnahmeeinrichtung verfügt über eine nichtschneidende Schabkante, durch die bei einer Biopsie die Entnahme von Material ohne einen Schneidvorgang erfolgt. Das im Körper des Patienten abgeschabte Material wird in den Innenraum der Entnahmeeinrichtung geleitet und kann so dem Körper entnommen werden.

US 5 573 008 A wird als der nächste Stand der Technik angesehen, und offenbart ein Instrument nach dem Oberberiff des Anspruchs 1.

Generell von großem Vorteil ist es, dass aufgrund der immer besser werdenden diagnostischen Möglichkeiten, bedingt vor allem durch qualitativ hochwertige bildgebende Verfahren, werden in vielen Fällen bereits vergleichsweise kleine Tumore, insbesondere Mammatumore, entdeckt. Problematisch an den bekannten technischen Lösungen ist in diesem Zusammenhang, dass die Entnahme derart kleiner Tumore mit den bekannten Vorrichtungen nicht zufriedenstellend erfolgen kann. Vor allem die Entnahme des vollständigen Tumors und damit eine möglichst exakte Aufarbeitung der Resektionsgrenzen lässt sich mit den bekannten Vorrichtungen nicht realisieren.

Ausgehend von den aus dem Stand der Technik bekannten Vorrichtungen und dem zuvor geschilderten Problemen ist es daher wünschenswert, eine Vorrichtung zur Entnahme von biologischem Material aus einem menschlichen oder tierischen Körper anzugeben, durch die auch vergleichsweise kleine Tumore, beispielsweise Mammatumore, die oftmals einen mittleren Durchmesser von bis zu 2 cm aufweisen, vollständig, möglichst lagetreu und deutlich gegenüber dem umliegenden, gesunden Gewebe abgegrenzt entnommen werden können. Es sollte somit möglich sein, das für die Entnahme vorgesehene biologische Material vollständig, also möglichst in einem Stück, dem Körper eines Patienten zu entnehmen und gleichzeitig die Belastung des umliegenden gesunden Gewebes und damit des Patienten gering zu halten. Hierbei sollte mit der anzugebenden Vorrichtung eine Tumor- oder Gewebeentnahme insbesondere derart durchzuführen sein, dass die Resektionsgrenzen in der Pathologie exakt aufgearbeitet werden können. Die anzugebende Vorrichtung sollte dabei die dem Anwender von anderen Instrumenten aus dem Stand der Technik vertraute Handhabung ermöglichen und über einen vergleichsweise einfachen konstruktiven Aufbau verfügen, wobei bevorzugt bereits bewährte Konstruktionsprinzipien zum Einsatz kommen sollten. Weiterhin sollte die anzugebende Vorrichtung zur Entnahme biologischen Materials auch eine bipolare Anwendung unter Einsatz der auf dem Markt verfügbaren Generatoren ermöglichen. Auch die Unterstützung einer Tumorentfernung durch bildgebende Verfahren, insbesondere durch geeignete Ultraschallverfahren und/oder Magnetresonanztomographie (MRT) sollte möglich sein.

Die zuvor genannte Aufgabe wird mit einem medizinischen Instrument gemäß Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft ein medizinisches Instrument zur minimalinvasiven Entnahme von biologischem Material aus einem menschlichen oder tierischen Körper. Das Instrument verfügt über eine Hohlnadel, die an einem einen Innenraum der Hohlnadel begrenzenden distalen Ende über einen Umfangsrand, der wenigstens abschnittsweise eine Schneidkante aufweist, und einen Schneiddraht, der derart angeordnet ist, dass der Schneiddraht zumindest teilweise außerhalb des Innenraums relativ zur Hohlnadel bewegbar ist, verfügt. Erfindungsgemäß ist das medizinische Instrument dahingehend weitergebildet worden, dass im Innenraum der Hohlnadel ein in Längsrichtung der Hohlnadel verschiebbares Führungselement vorgesehen ist, an dem der Schneiddraht zumindest an einer Stelle befestigt ist und dass ein Bewegungsmittel zur Bewegung des Schneiddrahtes vorgesehen ist, durch das eine Länge eines sich außerhalb des Innenraums befindenden Teils des Schneiddrahtes in Abhängigkeit der Betätigung eines Stellelements gezielt veränderbar ist. Erfindungswesentlich ist somit, dass das medizinische Instrument über einen Schneiddraht verfügt, der in das zu entfernende biologische Material und/oder das dieses Material, beispielsweise einen Tumor, umgebende Gewebe eingeführt werden kann und die Länge des außerhalb der Hohlnadel befindlichen Teils des Schneidrats verändert, insbesondere bedarfsgerecht angepasst werden kann. Mithilfe der erfindungsgemäßen technischen Lösung ist es daher möglich, den vom Schneiddraht zumindest teilweise umgebenden Schneidbereich, in dem ein für die Entnahme vorgesehenes biologisches Material, insbesondere ein Organ- oder Gewebeteil, liegt, auch während des Eingriffs zu verändern und an die jeweilige Situation anzupassen. Es ist somit möglich, vor allem bei zusätzlichem Einsatz geeigneter bildgebender Verfahren, wie etwa bildgebenden Ultraschallverfahren oder Magnetresonanztomographie (MRT), besonders zielgerichtet und unter weitgehender Schonung von gesundem Gewebe exakt das für die Entnahme vorgesehene biologische Material aus einem Körper zu entfernen. Der Schneiddraht ist hierbei sowohl im Innenraum der Hohlnadel angeordnet als auch an dem beweglichen Führungselement befestigt. Auf vorteilhafte Weise ist es somit möglich, das Führungselement in Längsrichtung der Hohlnadel über das distale Ende der Hohlnadel nach außen zu bewegen, wobei der Schneiddraht in die gleiche Richtung bewegt wird.

Erfindungsgemäß ist der Schneiddraht außerdem relativ zum Führungselement bewegbar und im Bereich des aus der Hohlnadel hinausstehenden Teils des Führungselements kann durch den Schneiddraht eine Schlinge oder Halbschlinge gebildet werden, die den Schneidbereich zumindest teilweise umgibt. Durch Bewegen des Schneiddrahts relativ zum Führungselement ist gemäß dieser Ausgestaltung der Erfindung, die Größe der Schlinge und damit des Schneidbereichs an die Größe des für die Entnahme vorgesehenen biologischen Materials anpassbar.

Grundsätzlich ist es denkbar, das erfindungsgemäß ausgebildete medizinische Instrument derart auszubilden, dass es entweder einmal oder mehrmals verwendbar ist. Auf vorteilhafte Weise lässt sich mit einem medizinischen Instrument gemäß der Erfindung biologisches Material, insbesondere Tumore, mit einem mittleren Durchmesser von bis zu 2 cm einem menschlichen oder tierischen Körper entnehmen. Von großem Vorteil hierbei ist, dass sich etwa auch Mammakarzinome in der Brust bereits in einem vergleichsweise frühen Stadium entfernen und/oder pathologisch untersuchen lassen. Dies ist wichtig, da aufgrund der immer besser werdenden bildgebenden Verfahren bereits sehr kleine Tumore erkannt und daraufhin entfernt werden.

Bevorzugt erfolgt die Entnahme von biologischem Material mit Hilfe des erfindungsgemäßen medizinischen Instruments unter Zuhilfenahme eines bildgebenden Verfahrens, beispielsweise eines bildgebenden Ultraschallverfahrens oder Magnetresonanztomographie, bei gleichzeitiger Lokalanästhesie. Der Einsatz geeigneter bildgebender Verfahren stellt hierbei eine sichere Navigation des Instruments gemäß der Erfindung innerhalb des Körpers eines Patienten sowie eine exakte, das umgebende Gewebe zumindest weitgehend schonende Resektion des für die Entnahme vorgesehenen biologischen Materials sicher. Vor allem zur Entfernung von Brusttumoren kann das erfindungsgemäß ausgebildete medizinische Instrument auf besonders vorteilhafte Weise eingesetzt werden.

Gemäß der Erfindung ist das Bewegungsmittel zur gezielten Bewegung des Schneiddrahtes derart ausgeführt, dass der Schneiddraht außerhalb des Innenraums der Hohlnadel zu einer zumindest teilweise einen Schneidbereich einschließenden Schneidschlaufe oder Schneidschlinge formbar ist, wobei eine Größe des Schneidbereiches in Abhängigkeit der Betätigung eines Betätigungselements und der hierdurch erfolgten Aktivierung eines Stellelements, das eine Wirkverbindung zwischen Betätigungselement und Bewegungsmittel herstellt, gezielt veränderbar ist. Dem Anwender, also einem Arzt, der einen minimalinvasiven Eingriff mit dem erfindungsgemäßen medizinischen Instrument vornimmt, ist es hierbei möglich, den Schneidbereich durch Betätigen des Betätigungselementes gezielt zu verändern, insbesondere auch während sich die Hohlnadeln bereits innerhalb des Körpers des Patienten, befindet. In diesem Zusammenhang ist es etwa denkbar, dass die Hohlnadel mit dem im Innenraum der Hohlnadel angeordneten Führungselement sowie dem daran befestigten Schneiddraht zunächst in den Patienten eingeführt wird und erst im Bereich des zu entfernenden biologischen Materials das Führungselement mit dem Schneiddraht aus dem Innenraum über das distale Ende hinaus aus der Hohlnadel ausgeschoben wird. Im Weiteren wird der Schneiddraht derart bewegt, dass eine Schneidschlaufe oder Schneidschlinge im Bereich des zu entfernenden biologischen Materials gebildet wird. Durch eine derart einstellbare Schneidschlinge kann die Resektionsgröße exakt eingestellt und bei Bedarf auch während des Eingriffs verändert werden.

Im Weiteren ist es auf vorteilhafte Weise denkbar, dass bereits beim Heranfahren des medizinischen Instruments an den Bereich des zu entfernenden biologischen Materials außerhalb des Innenraums der Hohlnadel die Schlinge zumindest teilweise ausgebildet wird, indem der Schneiddraht ausgefahren wird, sodass eine Rückzugstrasse, eine Öffnung oder ein Spalt erzeugt wird, durch den später das zu entfernende biologische Material aus dem Körper abgeführt werden kann. In diesem Zusammenhang ist es von Vorteil, wenn das Führungselement mit dem daran befestigten Schneiddraht zur Entfernung des biologischen Materials, insbesondere eines Tumor, mit seiner distalen Spitze mittig in das zu entfernende biologische Material eingestochen, durch dieses hindurchgeführt und etwa 3 mm hinter dem zu entfernenden biologischen Material zum Stehen gebracht wird. Bevorzugt erfolgt nun das Ausfahren des Schneiddrahtes, bevorzugt unter Ausbildung der Schneidschlinge, die bedarfsgerecht an die benötigte Resektionsgröße angepasst wird. Durch eine Drehbewegung um 360° oder um 180° in entgegengesetzte Richtungen wird daraufhin ein zumindest annähernd kugelförmiges Stück des zu entfernenden biologischen Materials ausgeschnitten.

Gemäß einer speziellen Ausführungsform ist es denkbar, auch während des Schneidvorgangs den Schneiddraht auf geeignete Weise zu bewegen, so dass die Form und/oder die Größe des Schneidbereichs und damit des ausgeschnittenen biologischen Materials verändert wird. So ist es etwas denkbar, dass kein kugelförmiges, sondern ein Stück mit wenigstens teilweise ovaler Form ausgeschnitten wird.

Ein besonders vorteilhaft ausgeführter minimalinvasiver Eingriff mit dem erfindungsgemäßen Instrument sieht vor, dass nach vollzogenem Schneidvorgang der Schneiddraht wieder etwas in den Innenraum der Hohlnadel zurückgezogen wird, sodass sich die außerhalb des Innenraums der Hohlnadel befindende Schneidschlinge verkürzt bzw. verkleinert und damit der Schneidbereich kleiner wird. Vorzugsweise halbiert sich hierbei die Größe des Schneidbereichs. Im Weiteren ist es, während die Hohlnadel aus dem Körper des Patienten gezogen wird, von Vorteil, wenn der Schneiddraht nicht vollständig in den Innenraum der Hohlnadel eingezogen wird, sondern bevorzugt in Form einer Schlinge im ausgeschnittenen biologischen Materials verbleibt, um das ausgeschnittene Material während der Bewegung durch den Körper des Patienten zu stabilisieren und so ein vergleichsweise einfaches Entfernen zu ermöglichen.

Gemäß einer weiteren besonderen Ausführungsform der Erfindung ist das Führungselement mit dem daran befestigten Schneiddraht im Innenraum der Hohlnadel in einer Hülse, die koaxial zur Hohlnadelwand angeordnet ist, gelagert und in Längsrichtung innerhalb der Hülse verschiebbar. Im Weiteren ist es von Vorteil, wenn das Führungselement eine Ausnehmung aufweist, die zumindest einen Teil des Schneiddrahtes aufnimmt. Denkbar ist es etwa, dass das Führungselement zylinder- oder rohrförmig ausgeführt ist und auf seiner Außenseite eine Kerbe aufweist, in der zumindest ein Teil des Schneiddrahtes angeordnet ist. Wird der wenigstens an einer Stelle am Führungselement befestigte Schneiddraht relativ zum Führungselement bewegt, so führt zumindest ein Abschnitt des Schneiddrahts eine Bewegung innerhalb der Ausnehmung in Längsrichtung des Führungselements aus.

Bei einer weiteren speziellen Ausführungsform ist auf einer Außenseite einer den Innenraum der Hohlnadel umfangsseitig umgebenden Hohlnadelwand wenigstens ein Dilatatorelement angeordnet, das zumindest bereichsweise relativ zur Hohlnadelwand aus einer Ruheposition in eine Eingriffsposition bewegbar ist. Vorzugsweise ist hierbei eine Oberfläche des Dilatatorelements in der Ruheposition zumindest annähernd parallel zu der die Außenseite der Hohlnadelwand begrenzenden Oberfläche angeordnet. Im Weiteren ist es von Vorteil, wenn das Dilatatorelement in Ruheposition wenigstens bereichsweise koaxial zur Hohlnadelwand angeordnet ist und wenigstens zwei in unterschiedliche Richtungen in die Eingriffsposition bewegbare Dilatatorarme aufweist. Mithilfe eines derartigen Dilatatorelements ist somit, insbesondere vor oder während die Hohlnadel aus ihrer Eingriffsposition aus dem Körper herausbewegt wird, im Körper ein Spalt oder eine Öffnung erzeugbar, sodass die Hohlnadel mit dem an der Hohlnadel befestigten biologischen Material vergleichsweise einfach aus dem Körper des Patienten durch das umgebende gesunde Gewebe bewegt werden kann. In einer ganz speziellen Weiterbildung verfügt das Dilatatorelement über vier, zumindest bereichsweise gezielt nach außen bewegbare Dilatatorarme oder -flügel.

Während des Zurückziehens der Hohlnadeln mit dem in die Eingriffsposition ausgefahrenen Dilatatorelement wird das die Hohlnadel umgebende Gewebe somit etwas weggedrückt und eine hierdurch vergrößerte Durchgangsöffnung für das bewegte biologische Material zur Verfügung gestellt. Vorzugsweise verfügt das Dilatatorelement über eine Mehrzahl von Armen, wobei es von besonderem Vorteil ist, wenn diese Dilatatorarme Teil einer Dilatatorhülse sind, die in Ruheposition koaxial zur Hohlnadelwand ausgerichtet ist und im Bedarfsfall gezielt vom Operateur aktivierbar ist. Das Dilatatorelement stellt somit sicher, dass im Körper des Patienten ein Rückweg dilatiert wird, um eine möglichst schonende Bewegung des ausgeschnittenen biologischen Materials durch das umliegende gesunde Gewebe zu realisieren.

Im Weiteren ist es denkbar, dass die für die Entnahme des biologischen Materials vorgesehene Durchgangsöffnung, etwa durch eine Drahtschlinge, insbesondere den erfindungsgemäß an der Hohlnadel angeordneten Schneiddraht oder durch eine sogenannte Messerinszision erweitert wird.

Eine ganz spezielle Ausgestaltung der Erfindung sieht vor, dass das Dilatatorelement über wenigstens einen Formgedächtniswerkstoff verfügt. Unter Formgedächtniswerkstoff wird in diesem Zusammenhang ein Werkstoff verstanden, der nach Verformung aus einer Ruheform in eine modifizierte Form, beispielsweise durch einen Wärmeeintrag, und anschließender Abkühlung auf die Ausgangstemperatur wieder in die Ruheform zurückkehrt.

Im Übrigen sind spezielle Formgebungen des Dilatatorelements, insbesondere der sich beim Dilatieren nach außen bewegenden Arme des Dilatatorelements, denkbar. So ist das Dilatatorelement, insbesondere seine Arme, am distalen Ende vorzugsweise nach innen, also in Richtung der Hohlnadel und/oder des Führungselements, gekrümmt, ganz besonders bevorzugt umgebogen. Auf diese Weise wird eine erleichterte Heranführung des medizinischen Instruments an das zu entfernende biologische Material ermöglicht. Ebenso ist es denkbar, das derart geformte Dilatatorelement und/oder seine Arme bei einer Bewegung des Schneiddrahts zumindest geringfügig zu dilatieren, um so eine leichte, stumpfe Dilatation zu erreichen.

Gemäß einer weiteren speziellen Ausgestaltung der Erfindung ist vorgesehen, dass das Dilatatorelement derart ausgeführt ist, dass das ausgeschnittene biologische Material von diesem ergriffen und während einer Bewegung durch das umliegende Gewebe fixiert wird. Vorteilhaft ist es in diesem Zusammenhang, wenn das Dilatatorelement eine Mehrzahl von Armen aufweist, die an ihren distalen Enden umgebogen sind und auf ihrer dem Führungselement zugewandten Seite einen Greifbereich aufweisen. Ein derart geformtes Dilatatorelement kann das ausgeschnittene Material wie eine Greifzange mit einem Greifmaul fixieren.

Im Weiteren ist in einer vorteilhaften Ausführungsform des erfindungsgemäßen Instruments ein über das distale Ende hinaus aus dem Innenraum heraus bewegbares Greifelement zur Fixierung des ausgeschnittenen biologischen Materials vorgesehen. Mit Hilfe eines derartigen Greifelements wird das ausgeschnittene biologische Material fixiert, um dieses möglichst vollständig und in seiner ursprünglichen Ausrichtung aus dem Körper entfernen können. In diesem Fall ist es denkbar, dass ein entsprechendes Greifelement derart ausgeführt ist, dass dieses nach vollendetem Schneidvorgang durch Betätigung eines Bedienelemente gezielt aus dem Innenraum der Hohlnadel über das distale Ende der Hohlnadel hinaus ausfahrbar ist. Im Folgenden werden bevorzugt geeignete Greifarme des Greifelements um das ausgeschnittene biologische Material gelegt, um dieses zu fixieren, aber ohne es zu beschädigen.

Alternativ oder ergänzend ist es denkbar, dass ein entsprechendes Greifelement zur Fixierung des ausgeschnittenen biologischen Materials nicht am distalen Ende der Hohlnadel, sondern wenigstens zeitweise am distalen Ende des medizinischen Instruments, das sich in der Eingriffsposition auf der der Hohlnadel gegenüberliegenden Seite des ausgeschnittenen biologischen Materials befindet, angeordnet ist.

Gemäß einer speziellen Weiterbildung der Erfindung ist das Greifelement wenigstens zum Teil relativ zum Führungselement bewegbar ausgeführt und über geeignete Bewegungsmittel an das medizinische Instrument angebunden. Bevorzugt ist das Greifelement teilweise im Innenraum der Hohlnadel zwischen der Hohlnadelwand und dem Führungselement angeordnet, wobei es grundsätzlich denkbar ist, dass das Greifelement von einer zur Hohlnadelwand konzentrischen Hülse umfangsseitig umgeben ist. Nachdem das ausgeschnittene biologische Material durch das Greifelement ergriffen worden ist, sind generell zwei unterschiedliche Szenarien für das weitere Vorgehen denkbar. Einerseits ist es möglich, dass das biologische Material in dieser Position, in der es durch das Greifelement fixiert ist, durch das umliegende gesunde Gewebe aus dem Körper entfernt wird. Andererseits ist es denkbar, dass das ausgeschnittene biologische Material mithilfe des Greifelementes in einen im Inneren der Hohlnadel vorgesehener Entnahmeraum überführt wird und hierin gemeinsam mit den übrigen Elementen des medizinischen Instrumentes aus dem Körper hinausbewegt wird. In diesem Fall ist auch das Führungselement mit dem daran befestigten Schneiddraht derart ausgebildet, dass im Innenraum der Hohlnadel ein ausreichender Platz vorhanden ist, um einen Entnahmeraum für das ausgeschnittene biologische Material bereitstellen zu können.

In einer weiteren Ausführungsform ist das Greifelement zumindest teilweise im Inneren des Führungselements angeordnet. In diesem Zusammenhang ist es denkbar, dass das Führungselement im Inneren über eine zylinderförmige Ausnehmung verfügt, in dem das Greifelement angeordnet ist, wobei das Greifelement besonders bevorzugt entlang der Längsachse des Führungselements bewegbar ist. Unabhängig von der Anordnung des Greifelements entweder am Ende des distalen Endes des Innenraums der Hohlnadel oder am Führungselement ist es des Weiteren von Vorteil, wenn das Greifelement wenigstens einen Formgedächtniswerkstoff aufweist. Auch in diesem Fall zeichnet sich der Formgedächtniswerkstoff dadurch aus, dass dieser nach Verformung aus einer Ruheform in eine modifizierte Form, beispielsweise durch einen Wärmeeintrag, und anschließender Abkühlung auf die Ausgangstemperatur wieder in die Ruheform zurückkehrt.

Eine weitere Ausgestaltung der Erfindung sieht vor, dass eine Betriebssituation des medizinischen Instruments herstellbar ist, in der der Schneiddraht mit elektrischer Energie versorgt wird und mit dem Führungselement, dem Greifelement, einem im Inneren der Hohlnadel angeordneten elektrisch leitfähigen Element, einer im Innenraum der Hohlnadel angeordneten Hülse oder der Hohlnadelwand ein Elektrodenpaar bildet, wobei zwischen den Elektroden ein Stromfluss initiiert wird, sodass sich ein Lichtbogen in diesem Bereich ausbildet. In einer derartigen Betriebssituation bildet der Schneiddraht auf vorteilhafte Weise die aktive Elektrode des Elektrodenpaares, wobei zwischen den Elektroden ein Stromfluss bewirkt wird, um einen Lichtbogen zu erzeugen und so den Schneidvorgang im biologischen Material zumindest zu unterstützen. Diese Betriebssituation stellt eine sogenannte bipolare Anwendung dar, bei der ein Strom zwischen den beiden Elektroden des medizinischen Instrumentes und damit lediglich in einem definierten Bereich des biologischen Materials fließt. Im Gegensatz zur monopolaren Technik fließt hierbei der Strom nicht großräumig durch den Körper des Patienten und eine neutrale Elektrode wird nicht benötigt.

Auf ganz besonders vorteilhafte Weise bildet die Hohlnadelwand oder eine im Innenraum der Hohlnadel angeordnete Hülse die Gegenelektrode zum die aktive Elektrode darstellenden Schneiddraht.

In einer ganz speziellen Ausführungsform der Erfindung ist vorgesehen, dass das medizinische Instrument über einen Kanal verfügt, in dem ein endoskopisches Instrument, das die Betrachtung eines in distaler Richtung vor dem medizinischen Instrument gelegenen Gewebebereichs ermöglicht. Vorzugsweise ist der Kanal derart ausgebildet, dass ein Endoskop während eines Eingriffs durch diesen in Längsrichtung bewegt, insbesondere sowohl in den Körper des Patienten hinein- als auch aus diesem herausgezogen werden kann. Bevorzugt ist der Kanal im Inneren der Hohlnadel, ganz besonders im Inneren des Führungselementes angeordnet und verfügt über eine distale Öffnung durch die eine Optikeinheit und/oder Kamera des Endoskops wenigstens teilweise in einen außerhalb des medizinischen Instruments gelegenen Gewebebereich bewegbar ist. Alternativ oder in Ergänzung ist es denkbar, dass wenigstens ein Endoskop, mit dem ein Operationsbereich dem Operateur visualisiert werden kann, integraler Bestandteil des medizinischen Instruments ist. Vorzugsweise ist ein derart ausgebildetes Endoskop, insbesondere seine Optikeinheit oder Kamera wenigstens bereichsweise an der Hohlnadel oder dem in Längsrichtung der Hohlnadel bewegbaren Führungselement befestigt.

Im Übrigen ist es von besonderem Vorteil, wenn ein erfindungsgemäß ausgeführtes medizinisches Instrument aus nicht magnetischen Werkstoffen hergestellt wird. Dies ist vor allem dann erforderlich, wenn das medizinische Instrument gemäß der Erfindung unter Zuhilfenahme der Magnetresonanztomographie (MRT) als bildgebendes Verfahren zur Entnahme von biologischem Material aus einem menschlichen oder tierischen Körper eingesetzt wird.

Im Folgenden wird die Erfindung ohne Beschränkung des allgemeinen Erfindungsgedankens anhand von Ausführungsbeispiele unter Bezugnahme auf die Figuren näher erläutert. Dabei zeigen:
- Fig. 1:: Seitenansicht eines erfindungsgemäß ausgeführten medizinischen Instruments zur Entnahme von biologischem Material aus einem menschlichen oder tierischen Körper;
- Fig. 2:: Draufsicht auf das distale Ende einer Hohlnadel eines erfindungsgemäß ausgeführten medizinischen Instruments sowie
- Fig. 3:: Ausschnitt einer schematischen Schnittdarstellung der Hohlnadel eines erfindungsgemäß ausgeführten medizinischen Instruments.

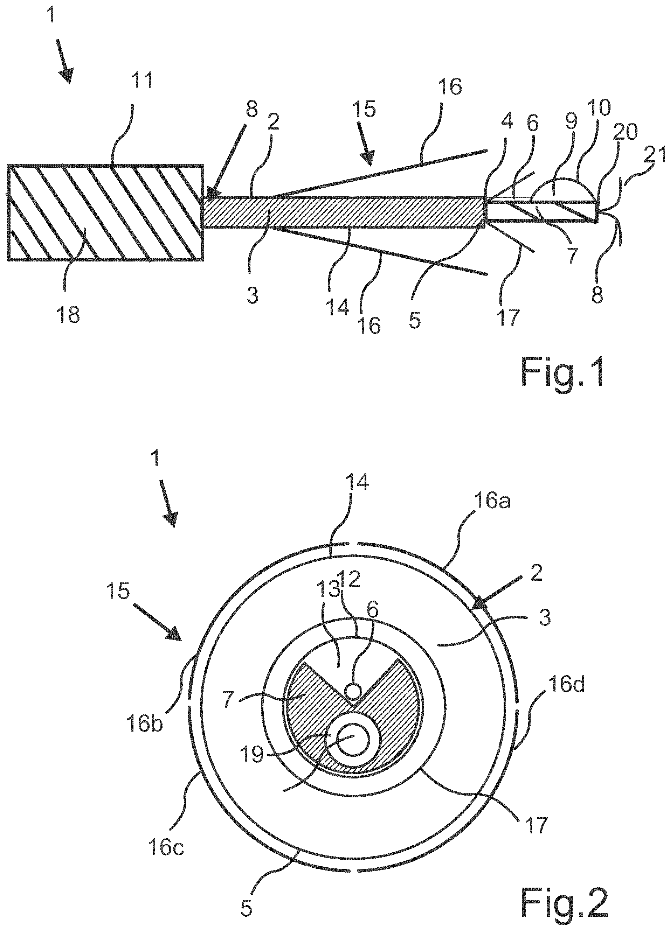

Fig. 1 zeigt in einer Seitenansicht ein medizinisches Instrument 1 zur Entfernung von biologischem Material, insbesondere eines Tumors, etwa eines Mammakarzinoms, aus einem menschlichen oder tierischen Körper. Das Instrument 1 verfügt über ein Griffstück 18, an dem ein Operateur, also insbesondere ein den Eingriff durchführender Arzt, das Instrument 1 erfassen, auf geeignete Weise bewegen und durch Betätigung wenigstens eines Stellelements 11 unterschiedlich Bedienfunktionen initiieren kann. Im Bereich des Griffstücks 12 sind somit für die Bedienung vorgesehene Stellelemente vorgesehen, durch die der Operateur verschiedene Elemente und Bewegungsmittel 8 des medizinischen Instruments 1 betätigten und insbesondere sowohl ein Schneidwerkzeug, das hier als an einem ebenfalls bewegbaren Führungselement 7 befestigter Schneiddraht 6 ausgeführt ist, ein Greifelement 17 zum Fixieren des ausgeschnittenen biologischen Materials als auch ein Dilatatorelement 15 bewegen kann.

Gemäß dem in Fig. 1 gezeigten Ausführungsbeispiel verfügt das medizinische Instrument 1 über eine hohlzylinder- oder hülsenförmig ausgebildete Hohlnadel 2, in deren Innenraum 3 ein in Längsrichtung der Hohlnadel 2 beweglich angeordnetes Führungselement 7 vorgesehen ist. Bei dem in Fig. 1 dargestellten Betriebszustand befindet sich das Führungselement 7 in einer Position, in der das Führungselement 7 über das distale Ende 4 des Innenraums 3 der Hohlnadel 3 hinausragt. Ferner ist am distalen Ende 4 des Führungselements 7 ein aufgrund manueller Betätigung eines Stellelements 11 und des hierdurch aktivierten Bewegungsmittels 8 bewegbarer Schneiddraht 6 befestigt. Im Übrigen ist der Schneiddraht 6 in einer als Kerbe ausgeführten Ausnehmung 13, die sich im Führungselement 7 in dessen Längsrichtung erstreckt angeordnet und relativ zum Führungselement 7 bewegbar, sodass außerhalb des Innenraums 3 der Hohlnadel 2 Schneidschlinge 10, die einen Schneidbereich 9 wenigstens teilweise umgibt, gebildet werden kann. Hierzu sind am Führungselement 7 geeignete Halte- und/oder Umlenkmittel vorgesehen, die derart auf den Schneiddraht 6 einwirken, dass bei einer Bewegung des Schneiddrahts 6 entlang des Führungselementes 7 in Richtung auf das distale Ende 20 des Führungselements 7 nicht nur die Länge des sich außerhalb des Innenraums 3 befindenden Schneiddrahts 6 verlängert wird, sondern auch eine geeignete Schneidschlinge 10 gebildet wird. Dieser Vorgang wird vom Operateur durch Betätigung wenigstens eines Stellelements 11 im Bereich des Greifelements 17 kontrolliert durchgeführt.

Gemäß dem in Fig. 1 gezeigten Ausführungsbeispiel lässt sich somit durch Bewegen des Schneiddrahtes 6 eine Schneidschlinge 10 außerhalb des Innenraums 3 der Hohlnadel 2 erzeugen, die als Schneidelement verwendet wird. Die Größe dieser Schneidschlinge 10 und damit des von dieser Schlinge 10 wenigstens teilweise eingeschlossenen Schneidbereichs 9 lässt sich vom Operateur bedarfsgerecht anpassen.

Bevorzugt erfolgt die Entfernung von biologischem Material, insbesondere eines Tumors mit einem mittleren Durchmesser von bis zu 2 cm aus der weiblichen Brust, unter Zuhilfenahme eines bildgebenden Verfahrens, beispielsweise eines bildgebenden Ultraschallverfahrens, einer Mammografie oder einer Magnetresonanztomographie (MRT) oder zukünftig nutzbarer bildgebender Verfahren, wie etwa Tomosynthese oder Rotations-Computertomographie, sodass der Operateur sicher und zielgerichtet den Tumor erreichen und mit hoher Genauigkeit aus dem gesunden Gewebe ausschneiden kann. Im Weiteren verfügt das in Fig. 1 gezeigte medizinische Instrument 1 an der Außenseite der Hohlnadelwand 14 über ein Dilatatorelement 15 in Form einer Hülse mit vier Dilatatorarmen 16, deren distale Enden jeweils ausklappbar sind. Sobald die Hohlnadel 2 mit dem ausgeschnittenen biologischen Material aus einem menschlichen oder tierischen Körper entfernt werden soll, kann der Operateur wiederum über ein Stell- oder Bedienelement die Dilatatorarme 16 betätigen, sodass sich diese mit ihren distalen Enden jeweils nach außen bewegen und eine Keilform erzeugen, sodass das ausgeschnittene biologische Material durch eine vergleichsweise große Durchgangsöffnung durch das umgebende gesunde Gewebe durch den Körper bewegt und diesem schließlich entnommen werden kann.

Weiterhin ist am distalen Ende 4 der Hohlnadel 2 ein Greifelement 17 mit vier Greifarmen angeordnet, die, nachdem das zu entnehmende biologische Material ausgeschnitten wurde, um das ausgeschnittene Material gelegt werden, sodass sichergestellt wird, dass das biologische Material möglichst vollständig und in der ursprünglichen Position, die es im Körper innehatte, dem Körper entnommen werden kann. Außerdem ist gemäß dem in Fig. 1 gezeigten Ausführungsbeispiel am distalen Ende 20 des Führungselements 7 ein Ankerelement 21 vorgesehen, das in einer Ruheposition im Inneren des Führungselementes 7 gelagert ist, und dann, wenn die Hohlnadel 2 sowie das Führungselement 7, wie im Folgenden noch näher erläutert werden wird, sich in der Eingriffsposition im Körper befinden, aus dem Führungselement 7 ausgefahren wird. Das Ankerelement 21 fixiert die Hohlnadel 2 mit dem Führungselement 7 während des Schneidvorgangs in der Eingriffsposition.

Zur Entfernung von biologischem Material aus einem menschlichen oder tierischen Körper, insbesondere eines Tumors, beispielsweise eines Mammakarzinoms, wird die Hohlnadel 2 mit dem sich in Ruheposition im Innenraum 3 der Hohlnadel 2 befindlichen Führungselement 7 und dem daran einseitig befestigten Schneiddraht 6, unter Zuhilfenahme eines bildgebenden Verfahrens, etwa eines bildgebenden Ultraschalldiagnostikverfahrens, in die Brust eingestochen. In unmittelbarer Nähe des zu entfernenden Tumors, wird das Führungselement 7 mit dem daran befestigten Schneiddraht 6 aus dem Innenraum der Hohlnadel 2 ausgefahren. Ebenso ist es denkbar, bereits beim Anfahren des Tumors, das Führungselement 7 und den Schneiddraht 6 auszufahren und so mithilfe des Schneiddrahts 6, insbesondere einer geformten Schneidschlinge 10, bereits eine erweiterte Durchgangsöffnung bzw. einen Spalt zu erzeugen, durch den später die Hohlnadel 2 mit dem ausgeschnittenen biologischen Material aus dem Körper entfernt werden kann.

Schließlich wird das Führungselement 7 mit dem Schneiddraht 6 mittig in den Tumor eingestochen, durch diesen hindurchgeschoben und die Bewegung gestoppt, sobald sich das distale Ende 20 des Führungselements 7, also die Spitze des medizinischen Instruments 1, etwa 3 mm hinter dem Tumor befindet. Nunmehr wird der Schneiddraht 6 derart bewegt, dass sich eine Schneidschlinge 10 mit der exakt benötigten Größe ausformt, es wird also der benötigte Radius eingestellt. Der mittlere Durchmesser des ausgeschnittenen biologischen Materials ist hierbei üblicherweise etwa 1 cm größer als der eines zu entfernenden Tumors. Auf diese Weise lässt sich die Resektionsgröße durch Verschieben des Schneiddrahts 6 und entsprechendes Ausformen einer Schneidschlinge 10 exakt einstellen. Durch eine Drehbewegung entweder um 360° oder um 180° jeweils in entgegengesetzter Richtung erfolgt schließlich das Ausschneiden von biologischem Material in Form einer Kugel. Es ist sogar möglich, dass der Operateur bei Bedarf auch während des Schneidvorgangs den Schneiddraht 6 in Längsrichtung des Führungselements 7 bewegt und so den außerhalb des Innenraums 3 der Hohlnadel 3 befindlichen Teil des Schneiddrahtes 6 und damit die Größe der Schneidschlinge 10 verändert. In diesem Fall nimmt das ausgeschnittene Material eine von der Kugelform abweichende Form an.

Nachdem das für die Entnahme vorgesehene biologische Material, hier ein Tumor in der weiblichen Brust, ausgeschnitten ist, wird der Schneiddraht 6 derart in den Innenraum 3 der Hohlnadel 2 eingezogen, dass sich die Grüße des Schneidbereichs 9 und damit der Schneidschlinge 10 etwa halbiert. Diese vergleichsweise kleine Schneidschlinge 10 verbleibt in dem zu entnehmenden Tumor, um diesen auf seiner Längsachse zu stabilisieren und eine sichere und einfache Entnahme zu gewährleisten. Bevor der Tumor durch den Körper bewegt wird, wird zunächst ein ebenfalls im Innenraum 3 der Hohlnadel 2 angeordnetes Greifelement 17, insbesondere dessen vierarmige Greifkralle, ausgefahren und der ausgeschnittene Tumor mithilfe der Greifkrallen fixiert. Im Anschluss daran wird der ausgeschnittene Tumor vorsichtig aus dem Körper herausbewegt. Um diesen Vorgang zu erleichtern, insbesondere um eine möglichst große Durchgangsöffnung für den zu entnehmenden Tumor bereitzustellen, wird vor dem Zurückziehen das Dilatatorelement 15 in Form einer vierarmigen, dilatierenden Hülse betätigt, sodass die einzelnen Dilatatorarme 16 nach außen bewegt werden. Auf diese Weise wird ein Keil geschaffen, der eine weitere Erleichterung bei der Entnahme des Tumors schafft. Um eine möglichst schonende Tumorentfernung durch das umliegende gesunde Gewebe zu realisieren, ist es denkbar, dass die Durchgangsöffnung entweder bereits beim Anfahren des Tumors mithilfe einer Schneidschlinge 10 erweitert wird. Auf bevorzugte Weise ist eine erfindungsgemäß bereitgestellte und genutzte Schneidschlinge 10 mit elektrischer Energie versorgbar und bildet gemeinsam mit einer weiteren Elektrode, etwa die Hohlnadelwand 14 oder ein sonstiges Element im Innenraum 3 der Hohlnadel 2 ein Elektrodenpaar, zwischen denen ein den Schneidvorgang unterstützender Lichtbogen gebildet wird. Im Übrigen ist es denkbar die Öffnung zur Entnahme des biologischen Materials mit Hilfe einer Messerinszision zu erweitern.

Fig. 2 zeigt in einer Draufsicht auf das distale Ende 4 einer Hohlnadel 2 eines erfindungsgemäß ausgeführten medizinischen Instruments 1. Deutlich zu erkennen ist, dass die einzelnen Komponenten, wie der Schneiddraht 6, die Hohlnadelwand 14, eine Führungshülse 12 zur Aufnahme des Führungselementes 7 sowie das Führungselement 7 mit dem Schneiddraht 6 und dem innenliegend angeordneten Ankerelement 21 konzentrisch um eine Längsmittenachse der Hohlnadel 2 angeordnet sind. Gemäß dem in Fig. 2 gezeigten Ausführungsbeispiel umschließt die Hohlnadelwand 14 umfangsseitig einen Innenraum 3, in dem sich eine weitere Hülse, die sogenannte Führungshülse 12, in Längsrichtung erstreckt. Innerhalb dieser Führungshülse 12 ist das Führungselement 7 in Längsrichtung bewegbar angeordnet. Ferner verfügt das Führungselement 7 über eine in Längsrichtung verlaufende Ausnehmung 13 in Form einer Kerbe, in der der als Schneidelement verwendete Schneiddraht 6 angeordnet ist. Im Weiteren verfügt das Führungselement 7 über eine zylinderförmige, sich ebenfalls in Längsrichtung erstreckende Bohrung 19, in der ein Ankerelement 21 angeordnet ist.

Auf der Außenseite der Hohlzylinderwand 14 befindet sich ein Dilatatorelement 15, das über vier parallel zur Außenfläche der Hohlzylinderwand angeordnete und parallel hierzu geformte Dilatatorarme 16a, 16b, 16c, 16d verfügt, die bewegbar und beim Herausziehen der Hohlnadel 2 aus dem Körper des Patienten zumindest bereichsweise von der Hohlnadelwand 14 entfernbar, also dilatierbar sind. Auf diese Weise lässt sich bedarfsgerecht ein Keil erzeugen, der bei einer Bewegung der Hohlnadel 2 in proximaler Richtung das umgebende, üblicherweise gesunde Gewebe wegdrückt und so die Durchgangsöffnung zur Hindurchführung der Hohlnadel 2 mit dem ausgeschnittenen Tumor vergrößert.

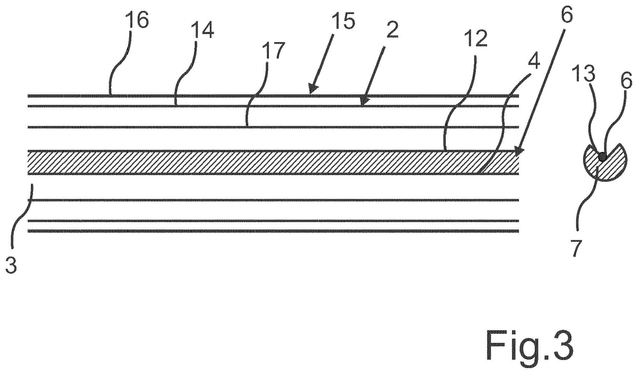

Fig. 3 zeigt im Weiteren in einer schematischen Schnittdarstellung die Hohlnadel 2 eines erfindungsgemäß ausgeführten medizinischen Instruments 1 mit den übrigen in diesem Bereich vorgesehenen Komponenten. Im Innenraum 3 der Hohlnadel 2 befindet sich wiederum das Führungselement 7, das in einer Führungshülse 12 angeordnet ist. Das Führungselement 7 verfügt über eine Längsrichtung verlaufende Ausnehmung 13 in Form einer Kerbe zur Aufnahme des Schneiddrahts 6, der das Schneidelement bildet.

Sowohl der Schneiddraht 6 als auch die Führungshülse 12, innerhalb der das Führungselement 7 angeordnet ist, sind elektrisch leitfähig mit einer Stromversorgung verbunden und bilden in einer speziellen Betriebssituation, bei einer sogenannten bipolaren Anwendung, ein Elektrodenpaar, von dem der Schneiddraht 6 die aktive Elektrode darstellt. Nach Anlegen eines Stromes geeigneter Stärke wird ein Stromfluss zwischen diesen beiden Elektroden initiiert und ein Lichtbogen bildet sich aus, durch den der Schneidvorgang zumindest unterstützt werden kann. Gegenüber den bekannten monopolaren Anwendungen der HF-Chirurgie hat die bipolare Anwendung vor allem die Vorteile, dass der Stromfluss lokal im Körper des Patienten begrenzt ist und keine Neutralelektrode benötigt wird.

Weiterhin ist ein Greifelement 17 mit einer Mehrzahl sich in Längsrichtung der Hohlnadel 2 erstreckenden Greifarmen vorgesehen, die die Führungshülse 12 umfangsseitig umgeben und hierbei zwischen der Führungshülse 12 und der Hohlnadelwand 14 angeordnet sind. An dem distalen Ende dieses Greifelements 17 mit mehreren Greifarmen befinden sich Greifkrallen, die der Fixierung des ausgeschnittenen biologischen Materials dienen.

Auf der Außenseite der Hohlnadelwand 14 und koaxial hierzu befindet sich ein Dilatatorelement 15, das über einzelne Dilatatorarme 16 verfügt, die sich bei Entnahme des ausgeschnittenen biologischen Materials aus dem Körper abschnittsweise nach außen bewegen und so eine Durchgangsöffnung erzeugen, durch die das ausgeschnittene biologische Material vergleichsweise einfach und in einem Stück entnommen werden kann.

### Bezugszeichenliste

- 1: Medizinisches Instrument
- 2: Hohlnadel
- 3: Hohlnadelinnenraum
- 4: distales Ende der Hohlnadel
- 5: Umfangsrand
- 6: Schneiddraht
- 7: Führungselement
- 8: Bewegungsmittel
- 9: Schneidbereich
- 10: Schneidschlinge
- 11: Stellelement
- 12: Führungshülse
- 13: Ausnehmung
- 14: Hohlnadelwand
- 15: Dilatatorelement
- 16: Dilatatorarme
- 17: Greifelement
- 18: Griffstück
- 19: Bohrung im Führungselement
- 20: distales Ende des Führungselementes
- 21: Ankerelement

## Patentansprüche

1. Medizinisches Instrument (1) zur minimal invasiven Entnahme von biologischem Material aus einem menschlichen oder tierischen Körper mit einer Hohlnadel (2), die an einem einen Innenraum (3) der Hohlnadel (2) begrenzenden distalen Ende (4) über einen Umfangsrand (5), der wenigstens abschnittsweise eine Schneidkante aufweist, verfügt und mit einem Schneiddraht (6), der derart angeordnet ist, dass der Schneiddraht (6) zumindest teilweise außerhalb des Innenraums (3) relativ zur Hohlnadel (2) bewegbar ist, wobei im Innenraum (3) der Hohlnadel (2) ein in Längsrichtung der Hohlnadel (2) verschiebbares Führungselement (7) vorgesehen ist, an dem der Schneiddraht (6) zumindest an einer Stelle befestigt ist und **dadurch gekennzeichnet, dass** ein Bewegungsmittel (8) zur Bewegung des Schneiddrahts (6) relativ zum Führungselement (7) vorgesehen ist, durch das eine Länge eines sich außerhalb des Innenraums (3) befindenden Teils des Schneiddrahts (6) in Abhängigkeit der Betätigung eines auf das Bewegungsmittel (8) einwirkenden Stellelements (11) gezielt derart veränderbar ist, dass im Bereich des aus der Hohlnadel (2) hinausstehenden Teils des Führungselements (7) durch den Schneiddraht (6) eine Schlinge oder Halbschlinge erzeugbar ist, die einen Schneidbereich zumindest teilweise umgibt.

2. Medizinisches Instrument (1) nach Anspruch 1,
**dadurch gekennzeichnet, dass** das Bewegungsmittel (8) derart ausgeführt ist, dass der Schneiddraht (6) außerhalb des Innenraums (3) der Hohlnadel (2) zu einer zumindest teilweise einen Schneidbereich (9) einschließenden Schneidschlinge (10) formbar ist, wobei eine Größe des Schneidbereichs (9) in Abhängigkeit der Betätigung des Stellelements (11) gezielt veränderbar ist.

3. Medizinisches Instrument (1) nach Anspruch 1 oder 2,
**dadurch gekennzeichnet, dass** das Führungselement (7) mit dem daran befestigten Schneiddraht (6) in einer Führungshülse (12) im Innenraum (3) der Hohlnadel (2) angeordnet ist.

4. Medizinisches Instrument (1) nach Anspruch 3,
**dadurch gekennzeichnet, dass** das Führungselement (7) wenigstens abschnittsweise eine Ausnehmung (13) aufweist, in der zumindest ein Teil des Schneiddrahtes (6) angeordnet ist.

5. Medizinisches Instrument (1) nach einem der vorangehenden Ansprüche,
**dadurch gekennzeichnet, dass** auf einer Außenseite einer den Innenraum der Hohlnadel (2) umfangsseitig umgebenden Hohlnadelwand (14) wenigstens ein Dilatatorelement (15) angeordnet ist, das zumindest bereichsweise relativ zur Hohlnadelwand (14) aus einer Ruheposition in eine Eingriffsposition bewegbar ist.

6. Medizinisches Instrument (1) nach Anspruch 5,
**dadurch gekennzeichnet, dass** eine Oberfläche des Dilatatorelements (15) in der Ruheposition zumindest annähernd parallel zu einer die Außenseite der Hohlnadelwand (14) begrenzenden Oberfläche angeordnet ist.

7. Medizinisches Instrument (1) nach Anspruch 5 oder 6,
**dadurch gekennzeichnet, dass** das Dilatatorelement (15) in Ruheposition hülsenförmig und koaxial zur Hohlnadelwand (14) angeordnet ist und wenigstens zwei in unterschiedliche Richtungen in die Eingriffsposition bewegbare Dilatatorarme (16a, 16b) aufweist.

8. Medizinisches Instrument (1) nach einem der Ansprüche 5 bis 7,
**dadurch gekennzeichnet, dass** das Dilatatorelement (15) wenigstens einen Formgedächtniswerkstoff aufweist.

9. Medizinisches Instrument (1) nach einem der vorangehenden Ansprüche,
**dadurch gekennzeichnet, dass** ein über das distale Ende (4) hinaus aus dem Innenraum (3) herausbewegbares Greifelement (17) zur Fixierung des biologischen Materials vorgesehen ist.

10. Medizinisches Instrument (1) nach Anspruch 9,
**dadurch gekennzeichnet, dass** das Greifelement (17) relativ zum Führungselement (7) bewegbar angeordnet ist.

11. Medizinisches Instrument (1) nach Anspruch 9 oder 10,
**dadurch gekennzeichnet, dass** das Greifelement (17) wenigstens teilweise im Inneren des Führungselementes (7) angeordnet ist.

12. Medizinisches Instrument (1) nach Anspruch 9 oder 10,
**dadurch gekennzeichnet, dass** das Greifelement teilweise im Innenraum der Hohlnadel zwischen der Hohlnadelwand und dem Führungselement angeordnet ist.

13. Medizinisches Instrument (1) nach einem der Ansprüche 9 bis 12,
**dadurch gekennzeichnet, dass** das Greifelement (17) wenigstens einen Formgedächtniswerkstoff aufweist.

14. Medizinisches Instrument (1) nach einem der vorangehenden Ansprüche,
**dadurch gekennzeichnet, dass** eine Betriebssituation herstellbar ist, in der der Schneiddraht (6) mit elektrischer Energie versorgt wird und mit dem Führungselement (7), dem Greifelement (17), einem im Innenraum der Hohlnadel (2) angeordneten elektrisch leitfähigen Element, einer im Innenraum (3) der Hohlnadel (2) angeordneten Hülse oder der Hohlnadelwand (14) ein Elektrodenpaar bildet.

15. Medizinisches Instrument (1) nach einem der vorangehenden Ansprüche ausschließlich hergestellt aus nicht magnetischen Werkstoffen.

## Claims

1. A medical instrument (1) for the minimally invasive removal of biological material from a human or animal body with a hollow needle (2), which, at a distal end (4) delimiting an interior (3) of the hollow needle (2), includes a peripheral edge (5) having a cutting edge at least in sections, and with a cutting wire (6), which is arranged such that the cutting wire (6) can be moved relative to the hollow needle (2) at least partially outside the interior (3),
wherein in the interior (3) of the hollow needle (2), a guide element (7), slidable in the longitudinal direction of the hollow needle (2), is provided, at which the cutting wire (6) is fastened at least at one point, and **characterized in that** a means of movement (8) for moving the cutting wire (6) relative to the guide element (7) is provided, through which a length of a part of the cutting wire (6) located outside the interior (3) can be changed in a targeted manner as a function of the actuation of a control element (11) acting on the means of movement (8), such that in the area of the part of the guide element (7) protruding from the hollow needle (2) a loop or half-loop can be created by the cutting wire (6), which at least partially surrounds a cutting area.

2. The medical instrument (1) according to claim 1,
**characterized in that** the means of movement (8) is designed such that the cutting wire (6) outside the interior (3) of the hollow needle (2) can be shaped into a cutting loop (10) at least partially enclosing a cutting area (9), wherein a size of the cutting area (9) can be changed in a targeted manner as a function of the actuation of the control element (11).

3. The medical instrument (1) according to claims 1 or 2,
**characterized in that** the guide element (7) with the cutting wire (6) fastened thereon is arranged in a guide sleeve (12) in the interior (3) of the hollow needle (2).

4. The medical instrument (1) according to claim 3,
**characterized in that** the guide element (7) has a recess (13), at least in sections, in which at least part of the cutting wire (6) is arranged.

5. The medical instrument (1) according to any one of the preceding claims,
**characterized in that**, on an outer side of a wall of the hollow needle (14) circumferentially enclosing the interior of the hollow needle (2), at least one dilator element (15) is arranged, which, at least in areas, can be moved relative to the wall of the hollow needle (14) from a rest position into an engagement position.

6. The medical instrument (1) according to claim 5,
**characterized in that** a surface of the dilator element (15) in the rest position is arranged at least approximately parallel to a surface delimiting the outer side of the wall of the hollow needle (14).

7. The medical instrument (1) according to claims 5 or 6,
**characterized in that** the dilator element (15) in its rest position is arranged sleeve-shaped and coaxially to the wall of the hollow needle (14) and has at least two dilator arms (16a, 16b) movable in different directions into the engagement position.

8. The medical instrument (1) according to any one of claims 5 to 7,
**characterized in that** the dilator element (15) comprises at least one shape-memory material.

9. The medical instrument (1) according to any one of the preceding claims,
**characterized in that** a gripping element (17), which can be moved out of the interior (3) beyond the distal end (4), is provided for fixation of the biological material.

10. The medical instrument (1) according to claim 9,
**characterized in that** the gripping element (17) is arranged in a movable manner relative to the guide element (7).

11. The medical instrument (1) according to claims 9 or 10,
**characterized in that** the gripping element (17) is arranged at least partially in the interior of the guide element (7).

12. The medical instrument (1) according to claims 9 or 10,
**characterized in that** the gripping element is partially arranged in the interior of the hollow needle, between the wall of the hollow needle and the guide element.

13. The medical instrument (1) according to any one of claims 9 to 12,
**characterized in that** the gripping element (17) comprises at least one shape-memory material.

14. The medical instrument (1) according to any one of the preceding claims,
**characterized in that** an operating situation can be created, in which the cutting wire (6) is supplied with electrical energy and forms a pair of electrodes with the guide element (7), the gripping element (17), an electrically conductive element arranged in the interior of the hollow needle (2), a sleeve arranged in the interior (3) of the hollow needle (2) or the wall of the hollow needle (14).

15. The medical instrument (1) according to any one of the preceding claims, exclusively manufactured from non-magnetic materials.

## Revendications

1. Instrument médical (1) pour le prélèvement mini-invasif de matière biologique dans un corps humain ou animal, comprenant une aiguille creuse (2) qui dispose, à une extrémité distale (4) délimitant un espace intérieur (3) de l'aiguille creuse (2), d'un bord périphérique (5) qui présente au moins par sections une arête de coupe, et comprenant un fil de coupe (6) qui est disposé de telle sorte que le fil de coupe (6) peut être déplacé au moins partiellement à l'extérieur de l'espace intérieur (3) par rapport à l'aiguille creuse (2),
un élément de guidage (7) déplaçable dans la direction longitudinale de l'aiguille creuse (2) étant prévu dans l'espace intérieur (3) de l'aiguille creuse (2), sur lequel le fil de coupe (6) est fixé au moins à un endroit, et ledit instrument médical étant **caractérisé en ce qu'**un moyen de déplacement (8) est prévu pour déplacer le fil de coupe (6) par rapport à l'élément de guidage (7), par lequel une longueur d'une partie du fil de coupe (6) se trouvant à l'extérieur de l'espace intérieur (3) peut être modifiée de manière ciblée en fonction de l'actionnement d'un élément de réglage (11) agissant sur le moyen de déplacement (8) de telle sorte que, dans la zone de la partie de l'élément de guidage (7) dépassant de l'aiguille creuse (2), une boucle ou une demi-boucle peut être produite par le fil de coupe (6), laquelle entoure au moins partiellement une zone de coupe.

2. Instrument médical (1) selon la revendication 1,
**caractérisé en ce que** le moyen de déplacement (8) est réalisé de telle sorte que le fil de coupe (6) peut être formé à l'extérieur de l'espace intérieur (3) de l'aiguille creuse (2) en une boucle de coupe (10) entourant au moins partiellement une zone de coupe (9), une taille de la zone de coupe (9) pouvant être modifiée de manière ciblée en fonction de l'actionnement de l'élément de réglage (11).

3. Instrument médical (1) selon la revendication 1 ou 2,
**caractérisé en ce que** l'élément de guidage (7), avec le fil de coupe (6) qui y est fixé, est disposé dans une douille de guidage (12) dans l'espace intérieur (3) de l'aiguille creuse (2).

4. Instrument médical (1) selon la revendication 3,
**caractérisé en ce que** l'élément de guidage (7) présente au moins par sections un évidement (13) dans lequel est disposée au moins une partie du fil de coupe (6).

5. Instrument médical (1) selon l'une des revendications précédentes,
**caractérisé en ce qu'**au moins un élément dilatateur (15) est disposé sur un côté extérieur d'une paroi d'aiguille creuse (14) entourant l'espace intérieur de l'aiguille creuse (2) sur sa périphérie, lequel élément dilatateur peut être déplacé au moins par zones par rapport à la paroi d'aiguille creuse (14) d'une position de repos dans une position d'engagement.

6. Instrument médical (1) selon la revendication 5,
**caractérisé en ce qu'**une surface de l'élément dilatateur (15) dans la position de repos est au moins approximativement parallèle à une surface délimitant le côté extérieur de la paroi d'aiguille creuse (14).

7. Instrument médical (1) selon la revendication 5 ou 6,
**caractérisé en ce que** l'élément dilatateur (15) est disposé en forme de manchon en position de repos et coaxialement à la paroi de l'aiguille creuse (14) et présente au moins deux bras dilatateurs (16a, 16b) mobiles dans des directions différentes vers la position d'engagement.

8. Instrument médical (1) selon l'une des revendications 5 à 7,
**caractérisé en ce que** l'élément dilatateur (15) comprend au moins un matériau à mémoire de forme.

9. Instrument médical (1) selon l'une des revendications précédentes,
**caractérisé en ce qu'**il est prévu un élément de préhension (17) pouvant être déplacé hors de l'espace intérieur (3) au-delà de l'extrémité distale (4) pour fixer la matière biologique.

10. Instrument médical (1) selon la revendication 9,
**caractérisé en ce que** l'élément de préhension (17) est disposé de manière mobile par rapport à l'élément de guidage (7).

11. Instrument médical (1) selon la revendication 9 ou 10,
**caractérisé en ce que** l'élément de préhension (17) est disposé au moins partiellement à l'intérieur de l'élément de guidage (7).

12. Instrument médical (1) selon la revendication 9 ou 10,
**caractérisé en ce que** l'élément de préhension est partiellement disposé à l'intérieur de l'aiguille creuse entre la paroi de l'aiguille creuse et l'élément de guidage.

13. Instrument médical (1) selon l'une des revendications 9 à 12,
**caractérisé en ce que** l'élément de préhension (17) comprend au moins un matériau à mémoire de forme.

14. Instrument médical (1) selon l'une des revendications précédentes,
**caractérisé en ce qu'**il est possible d'établir une situation de fonctionnement dans laquelle le fil de coupe (6) est alimenté en énergie électrique et forme une paire d'électrodes avec l'élément de guidage (7), l'élément de préhension (17), un élément électriquement conducteur disposé dans l'espace intérieur de l'aiguille creuse (2), une douille disposée dans l'espace intérieur (3) de l'aiguille creuse (2) ou la paroi (14) de l'aiguille creuse.

15. Instrument médical (1) selon l'une des revendications précédentes, fabriqué exclusivement à partir de matériaux non magnétiques.
